(19) Europäisches Patentamt
European Patent Office
Office européen des brevets

(11) Veröffentlichungsnummer: **0 039 811**
**A2**

(12) # EUROPÄISCHE PATENTANMELDUNG

(21) Anmeldenummer: 81103121.0

(22) Anmeldetag: 25.04.81

(51) Int. Cl.³: **A 01 N 47/18**
A 01 N 43/82, A 01 N 43/78
A 01 N 43/76, A 01 N 43/52
A 01 N 43/50, C 07 D 453/06
C 07 D 417/12, C 07 D 413/12

(30) Priorität: 10.05.80 DE 3018075

(43) Veröffentlichungstag der Anmeldung:
18.11.81 Patentblatt 81/46

(84) Benannte Vertragsstaaten:
BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Förster, Heinz, Dr.
Am Eckbusch 47
D-5600 Wuppertal 1(DE)

(72) Erfinder: Hofer, Wolfgang, Dr.
Pahlkestrasse 59
D-5600 Wuppertal 1(DE)

(72) Erfinder: Maurer, Fritz, Dr.
Roeberstrasse 8
D-5600 Wuppertal 1(DE)

(72) Erfinder: Mues, Volker, Dr.
Gellertweg 13
D-5600 Wuppertal 1(DE)

(72) Erfinder: Eue, Ludwig, Dr.
Paul-Klee-Strasse 36
D-5090 Leverkusen(DE)

(72) Erfinder: Lürssen, Klaus, Dr.
August-Kierspel-Strasse 89
D-5060 Bergisch-Gladbach 2(DE)

(54) **Mittel zur Regulierung des Pflanzenwachstums, deren Herstellung und deren Verwendung.**

(57) Oxycarbonsäureamide der Formel

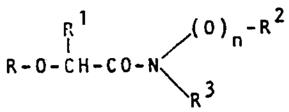

(1),

in welcher R, $R^1$, n, $R^2$ und $R^3$ die in der Beschreibung angegebene Bedeutung besitzen, lassen sich sehr gut zur Regulierung des Pflanzenwachstums verwenden.

EP 0 039 811 A2

Croydon Printing Company Ltd.

BAYER AKTIENGESELLSCHAFT 5090 Leverkusen, Bayerwerk
Zentralbereich
Patente, Marken und Lizenzen Dü/Kü-c
II b

# Mittel zur Regulierung des Pflanzenwachstums, deren Herstellung und deren Verwendung

Die vorliegende Erfindung betrifft die Verwendung von teilweise bekannten Oxycarbonsäureamiden als Wirkstoffe zur Regulierung des Pflanzenwachstums.

Es ist bereits bekannt geworden, daß bestimmte Oxycarbonsäureamide herbizide Eigenschaften besitzen (vgl. EP-OS 5 501).

Weiterhin ist bereits bekannt geworden, daß 2,3,5-Trijodbenzoesäure, 2-Chlorethyl-trimethylammonium-chlorid und 2-Chlor-ethanphosphonsäure pflanzenwuchsregulierende Aktivität besitzen (vgl. J. Agric. Food. Chem. 15 (1967), 976, US-PS 3 156 554 und Nature 218 (1968), 974). Allerdings ist die Wirksamkeit dieser Stoffe, vor allem bei niedrigen Aufwandmengen, nicht immer ganz befriedigend.

Weiterhin ist bekannt geworden, daß ein unter der Bezeichnung "Off-Shoot-T"(R) im Handel befindliches Produkt auf Basis von Fettalkoholen mit 6, 8, 10 und 12 Kohlenstoffatomen zur Regulierung des Pflanzenwachstums,

insbesondere zur Unterdrückung des Seitentriebwachstums bei Tabak eingesetzt werden kann (vgl. Farm. Chem. Handbook 1975, Meister Publishing Co., Willoughby, Ohio, 1975, Pesticide Dictionary D 147). Bei niedrigen Aufwandmengen werden aber die in der Praxis gestellten Forderungen bezüglich der Wirksamkeit nicht immer in ausreichendem Maße erfüllt.

Außerdem wurden auch schon bestimmte Aminoessigsäurederivate, die über Stickstoff mit einem Heterocyclus verknüpft sind, als Stoffe zur Regulierung des Pflanzenwachstums beschrieben (vgl. EP-OS 1349, EP-OS 3075, US-PS 4 075 216 und US-PS 4 160 658). Auch die Wirksamkeit dieser Stoffe läßt jedoch in manchen Fällen zu wünschen übrig.

Es wurde nun gefunden, daß die teilweise bekannten Oxycarbonsäureamide der Formel

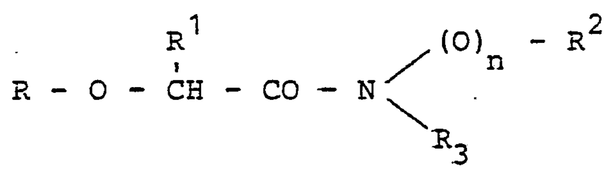

(I)

in welcher

R für einen fünfgliedrigen Heterocyclus steht, welcher ein Sauerstoff- oder ein Schwefelatom und zusätzlich 1 bis 3 Stickstoffatome enthält und welcher gegebenenfalls substituiert ist durch Halogen, Cyano, Nitro, Amino, Alkylamino, Arylamino, Dialkylamino,

Alkylcarbonylamino, Alkylcarbonyl, Carboxy, Alkoxycarbonyl, Carbamoyl, Alkylaminocarbonyl, Dialkylaminocarbonyl, gegebenenfalls durch Halogen, Nitro oder Alkyl substituiertes Arylaminocarbonyl, gegebenenfalls durch Halogen, Cyano, Nitro, Alkyl oder Alkoxy substituiertes Aryl, gegebenenfalls durch Halogen substituiertes Aralkyl, gegebenenfalls durch Halogen substituiertes Alkoxy, Alkenoxy, Alkinoxy, Alkoxycarbonylalkoxy, Aralkoxy oder Aryloxy, gegebenenfalls durch Halogen substituiertes Alkylthio, Alkenylthio, Alkinylthio, Alkoxycarbonylalkylthio, Aralkylthio oder Arylthio, gegebenenenfalls durch Halogen substituiertes Alkylsulfinyl oder Alkylsulfonyl, gegebenenfalls durch Halogen substituiertes Alkyl, Alkenyl, Alkinyl, Alkoxyalkyl, Aralkoxyalkyl, Aryloxyalkyl, Alkylthioalkyl, Alkylsulfinylalkyl, Alkylsulfonylalkyl, Arylthioalkyl, Arylsulfinylalkyl, Arylsulfonylalkyl, Carboxyalkyl, Alkoxycarbonylalkyl, gegebenenfalls substituiertes Aminocarbonylalkyl, Cyanoalkyl oder Cycloalkyl, oder welcher gegebenenfalls benzannelliert ist, wobei der Benzo-Rest gegebenenfalls durch Halogen, Alkyl, Halogenalkyl, Alkoxy, Halogenalkoxy, Alkylthio, Halogenalkylthio, Amino, Alkylamino, Dialkylamino, Nitro, Cyano, Alkoxycarbonyl oder durch gegebenenfalls halogen-substituiertes Alkylendioxy substitiert ist,

in welcher weiter

$R^1$ für Wasserstoff oder Alkyl steht,

n für Null oder 1 steht und

$R^2$ und $R^3$, welche gleich oder verschieden sein können, einzeln für Wasserstoff oder für gegebenenfalls substituierte Reste aus der Reihe Alkyl, Alkenyl, Alkinyl, Cycloalkyl, Cycloalkenyl, Aralkyl oder Aryl stehen oder, - für den Fall, daß n für Null steht -, zusammen mit dem Stickstoffatom an das sie gebunden sind, einen gegebenenfalls substituierten, gegebenenfalls teilweise ungesättigten und gegebenenfalls benzannelierten Mono- oder Bicyclus bilden, der gegebenenfalls weitere Heteroatome enthält,

starke pflanzenwachstumsregulierende Eigenschaften aufweisen.

Überraschenderweise zeigen die erfindungsgemäß verwendbaren Oxycarbonsäureamide der Formel (I) eine wesentlich bessere pflanzenwuchsregulierende Wirkung als die aus dem Stand der Technik bekannten Stoffe 2,3,5-Triiodbenzoesäure, 2-Chlorethyl-trimethyl-ammoniumchlorid und 2-Chlorethanphosphonsäure, welches hochaktive Stoffe gleicher Wirkungsart sind.

Außerdem übertreffen die erfindungsgemäßen Oxycarbonsäureamide auch die bekannten Aminoessigsäurederivate der Formeln

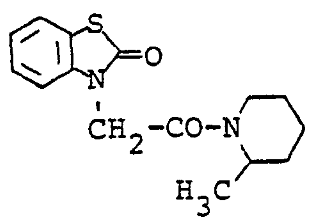
,
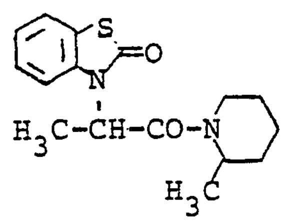
,
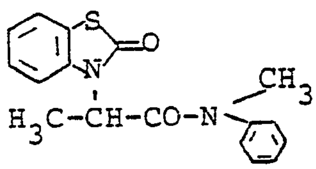
,
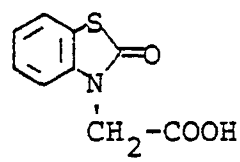
,
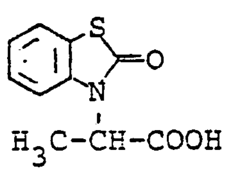
und
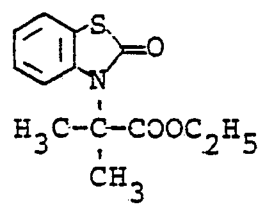

bezüglich ihrer pflanzenwuchsregulierenden Wirksamkeit. Die erfindungsgemäße Verwendung der Oxycarbonsäureamide der Formel (I) stellt somit eine wertvolle Bereicherung der Technik dar.

Die erfindungsgemäß zu verwendenden Oxycarbonsäureamide sind durch die Formel (I) allgemein definiert.

Vorzugsweise stehen in dieser Formel

R für den Rest

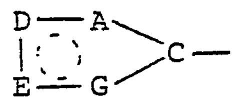

worin

A für $C-R^4$ oder N steht,

D für $C-R^5$ oder N steht,

E für $C-R^6$, N, O oder S steht und

G für $C-R^7$, N, O oder S steht,

mit der Maßgabe, daß wenigstens eins der Ringglieder A, D, E oder G für N steht und mindestens eins der Ringglieder E oder G für O oder S steht, wobei die Reste

$R^4$, $R^5$, $R^6$ und $R^7$,

welche gleich oder verschieden sein können, einzeln für Wasserstoff, Halogen, Nitro, Cyano, Amino, $C_1$-$C_4$-Alkylamino, Di-$C_1$-$C_4$-Alkylcarbonylamino, $C_1$-$C_4$-Alkyl-carbonyl, Carboxy, $C_1$-$C_4$-Alkoxy-carbonyl, Carbamoyl, $C_1$-$C_4$-Alkylamino-carbonyl, Di-$C_1$-$C_4$-alkyl-amino-carbonyl, für gegebenenfalls durch Halogen, Nitro oder $C_1$-$C_4$-Alkyl substituiertes Phenyl-amino-carbonyl, für gegebenenfalls durch Halogen, Nitro, Cyano, $C_1$-$C_4$-Alkyl

oder $C_1$-$C_4$-Alkoxy substituiertes Phenyl, für gegebenenfalls durch Halogen substituiertes Benzyl oder Phenylethyl, für gegebenenfalls durch Halogen substituiertes $C_1$-$C_4$-Alkoxy, $C_2$-$C_4$-Alkenoxy, $C_2$-$C_4$-Alkinoxy, $C_1$-$C_4$-Alkoxy-carbonylmethoxy, Benzyloxy oder Phenoxy, für gegebenenfalls halogen-substituiertes $C_1$-$C_4$-Alkylthio, $C_2$-$C_4$-Alkenylthio, $C_2$-$C_4$-Alkinylthio, $C_1$-$C_4$-Alkoxy-carbonyl-methylthio, Benzylthio, Phenylthio, $C_1$-$C_4$-Alkyl-sulfinyl oder $C_1$-$C_4$-Alkylsulfonyl, für gegebenenfalls halogen-substituiertes $C_1$-$C_6$-Alkyl, $C_2$-$C_6$-Alkenyl oder $C_2$-$C_6$-Alkinyl, für Cyano-$C_1$-$C_4$-alkyl, $C_1$-$C_4$-Alkoxy-$C_1$-$C_2$-alkyl, Phenoxy- und Phenylthio-methyl, Benzyloxy- und Benzylthiomethyl, $C_1$-$C_4$-Alkylthio-$C_1$-$C_2$-alkyl, $C_1$-$C_4$-Alkyl- und Phenylsulfinyl-$C_1$-$C_2$-alkyl, $C_1$-$C_4$-Alkyl- und Phenylsulfonyl-$C_1$-$C_2$-alkyl, Carboxy-$C_1$-$C_2$-alkyl, $C_1$-$C_4$-Alkoxy-carbonyl-$C_1$-$C_2$-alkyl, $C_1$-$C_4$-Alkylaminocarbonyl-$C_1$-$C_2$-alkyl, Di-$C_1$-$C_4$-Alkylaminocarbonyl-$C_1$-$C_2$-alkyl, Phenylamino-carbonyl-$C_1$-$C_2$-alkyl oder $C_3$-$C_{12}$-Cycloalkyl stehen,

oder wobei die Reste $R^5$ und $R^6$ zusammen mit den angrenzenden C-Atomen für einen anellierten Benzorest stehen, der durch Halogen, Nitro, Cyano oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiert sein kann;

weiter stehen in Formel (I) vorzugsweise

$R^1$ für Wasserstoff oder Methyl,

n für 0 oder 1,

$R^2$ für Alkyl, Alkoxyalkyl, Alkenyl oder Alkinyl, jeweils mit bis zu 10 Kohlenstoffatomen, und, - für den Fall, daß n für Null steht -, auch für Cyanoalkyl oder Alkylthioalkyl, jeweils mit bis zu 10 Kohlenstoffatomen, für Cycloalkyl mit 3 bis 12 Kohlenstoffatomen, für gegebenenfalls halogen-substituiertes Benzyl oder Phenethyl oder für Phenyl, welches gegebenenfalls durch halogen-substituierte Reste aus der Reihe $C_1$-$C_4$-Alkyl, $C_1$-$C_4$-Alkoxy oder $C_1$-$C_4$-Alkylthio substituiert ist,

und

$R^3$ für Alkyl, Alkenyl, Alkinyl, Alkoxyalkyl, Alkylthioalkyl, Cyanoalkyl, jeweils mit bis zu 10 Kohlenstoffatomen, für Cycloalkyl mit 3 bis 12 Kohlenstoffatomen, für gegebenenfalls halogensubstituiertes Benzyl, Phenethyl oder Naphthyl oder für Phenyl, welches gegebenenfalls durch Halogen, Cyano, Nitro oder durch gegebenenfalls halogen-substituierte Reste aus der Reihe $C_1$-$C_4$-Alkyl, $C_1$-$C_4$-Alkoxy oder $C_1$-$C_4$-Alkylthio substituiert ist,

und weiterhin stehen, - für den Fall, daß n für Null steht -, die Reste $R^2$ und $R^3$ zusammen mit dem Stickstoffatom, an das sie gebunden sind, für einen gegebenenfalls durch 1 bis 3 Alkylgruppen mit jeweils 1 bis 5 Kohlenstoffatomen oder durch zwei geminale Alkoxygruppen mit jeweils 1 bis 3 Kohlenstoffatomen substituierten oder gegebenenfalls durch Dioxolanyliden- oder Dioxanylidenreste spiro-cyclisch-verknüpft substituierten, gegebenenfalls teilweise ungesättigten und/oder benzannelierten Monocyclus oder Bicyclus mit bis zu 15 Kohlenstoffatomen, oder die Reste $R^2$ und $R^3$ bilden zusammen mit dem Stickstoffatom, an das sie gebunden sind, einen gegebenenfalls durch 1 bis 3 Alkylgruppen mit jeweils 1 bis 5 Kohlenstoffatomen, durch Phenyl, welches gegebenenfalls durch $C_1$-$C_4$-Alkyl, $C_1$-$C_4$-Alkoxy, Halogen, $C_1$-$C_2$-Halogenalkyl oder Nitro substituiert ist, durch Benzyl oder Phenylethyl substituierten, gesättigten und ein weiteres Stickstoffatom, Sauerstoffatom oder Schwefelatom enthaltenden Monocyclus mit bis zu 5 Kohlenstoffatomen.

Die Erfindung betrifft insbesondere die Verwendung von Verbindungen der Formel (I), in welcher R für einen der nachstehenden Azolylreste steht

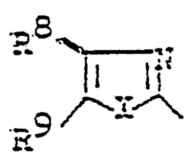 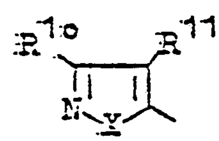 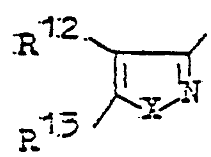

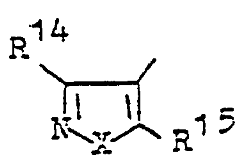 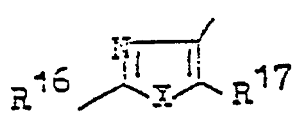 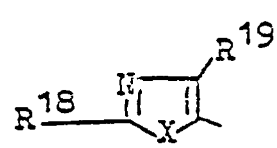

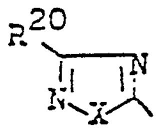

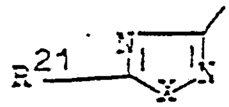

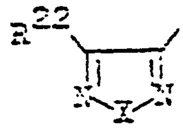

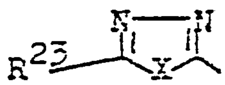

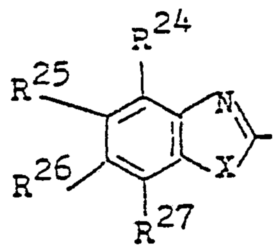

worin

X jeweils für Sauerstoff oder Schwefel steht, die Reste $R^8$ bis $R^{23}$, welche gleich oder verschieden sein können, einzeln für Wasserstoff, Brom, Chlor, Nitro, Cyano, $C_1$-$C_3$-Alkylcarbonyl, $C_1$-$C_3$-Alkoxycarbonyl, gegebenenfalls durch Fluor, Chlor oder Brom, Methyl, Methoxy, Nitro, Amino und/oder Cyano 1- oder 2fach substituiertes Phenyl, für Phenoxy oder Phenylthio, für $C_1$-$C_3$-Alkylthio oder $C_1$-$C_3$-Alkoxy, für $C_1$-$C_3$-Alkylsulfinyl oder $C_1$-$C_3$-Alkylsulfonyl, für $C_1$-$C_4$-Alkyl, Trifluormethyl, Cyano-$C_1$-$C_4$-Alkyl, $C_2$-$C_4$-Alkenyl, Benzyloxymethyl, $C_1$-$C_3$-Alkyl-amino, N-$C_1$-$C_3$-Alkyl-N-$C_1$-$C_4$-alkyl-carbonylamino, Phenoxymethyl, Benzylthio, oder für $C_1$-$C_3$-Alkyl-carbonyloxy stehen und

die Reste $R^{24}$ bis $R^{27}$, welche gleich oder verschieden sein können, einzeln für Chlor, $C_1$-$C_2$-Alkyl, $C_1$-$C_2$-Alkoxy, Trifluormethyl oder Trifluormethoxy stehen oder jeweils zwei benachbarte Reste zusammen für Methylendioxy, Dichlormethylendioxy oder Difluormethylendioxy stehen;

weiter betrifft die Erfindung insbesondere die Verwendung von Verbindungen der Formel (I), in welcher

$R^1$ für Wasserstoff oder Methyl steht,

n für 0 oder 1 steht,

$R^2$ für $C_1$-$C_6$-Alkyl, $C_1$-$C_4$-Alkoxy-ethyl, Allyl, Propargyl, 1-Methyl-propargyl oder 1,1-Dimethyl-propargyl oder, - für den Fall, daß n für Null steht -, auch für Cyanoethyl,, Cyclopentyl, Cyclohexyl, Benzyl oder Phenyl steht,

und

$R^3$ für $C_1$-$C_6$-Alkyl, $C_1$-$C_4$-Alkoxy-ethyl, Allyl, Propargyl, 1-Methyl-propargyl, 1,1-Dimethyl-propargyl, Cyanoethyl , Cyclopentyl, Cyclohexyl, Benzyl, Naphtyl oder Phenyl steht, welches gegebenenfalls durch Methyl, Chlor, Cyano, Nitro oder Methoxy substituiert ist, wobei auch mehrfache und gemischte Substitution durch die genannten Reste möglich ist, und weiterhin, - für den Fall, daß n für Null steht -, die Reste $R^2$ und $R^3$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl, Mono- oder Dialkylmorpholinyl mit 1 bis 3 Kohlenstoffatomen je Alkylgruppe, Piperidyl, Monoalkyl-, Dialkyl-, oder Trialkylpiperidyl mit 1 bis 3 Kohlenstoffatomen je Alkylgruppe, für 4,4-Dialkoxy-piperidyl mit 1 bis 3 Kohlenstoffatomen je Alkoxygruppe, für spirosubtituiertes Piperidyl der Formel

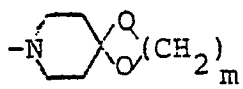

worin m für 2 oder 3 steht, für Perhydroazepinyl (Hexamethylenimino-Rest), Trimethyl-perhydroazepinyl, für den Heptamethylenimino-Rest, für den Dodekamethylenimino-Rest, für 1,2,3,4-Tetrahydroindolyl, für Monoalkyl-, Dialkyl- oder Trialkyl-1,2,3,4-tetrahydroindolyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinoyl, Monoalkyl-, Dialkyl- oder Trialkyl-1,2,3,4-tetrahydrochinolyl oder -isochinolyl mit 1 bis 3 Kohlenstoffatome je Alkylgruppe, für Perhydrochinolyl oder Perhydro-iso-chinolyl, Monoalkyl-, Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest der Formel

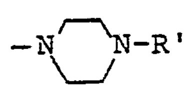

worin R' für $C_1$-$C_4$-Alkyl, für gegebenenfalls durch $C_1$-$C_2$-Alkyl, $C_1$-$C_2$-Alkoxy, Fluor, Chlor, Brom, Trifluormethyl und/oder Nitro substituiertes Phenyl, für Benzyl oder Phenylethyl steht, oder die Reste $R^2$ und $R^3$ zusammen mit dem Stickstoffatom, an das sie gebunden sind, für den Rest der Formel

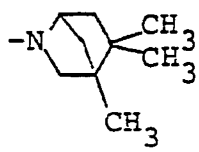

stehen.

Beispiele für die erfindungsgemäß verwendbaren Stoffe sind die in und nach den Herstellungsbeispielen aufgeführten Verbindungen.

Die erfindungsgemäß verwendbaren Oxycarbonsäureamide der Formel (I) sind zum Teil bereits bekannt (vgl. EP-OS 5 501). Die bisher noch nicht bekannten Verbindungen der Formel (I) sind Gegenstand von noch nicht veröffentlichten vorgängigen Patentanmeldungen. Die in diesen nicht vorveröffentlichten Patentanmeldungen angegebenen Verfahren zur Herstellung von Oxycarbonsäureamiden der Formel (I) sind im Prinzip mit dem in der EP-OS 5 501 beschriebenen Synthese-Verfahren identisch.

Man erhält demnach die Verbindungen der Formel (I), wenn man α-Hydroxy-carbonsäureamide der Formel

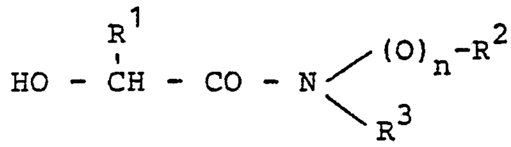 (II)

in welcher

$R^1$, $R^2$, $R^3$ und n die oben angegebenen Bedeutungen haben,

mit Halogenazolen der Formel

R - Hal (III)

in welcher

R die oben angegebene Bedeutung hat und

Hal für Chlor, Brom oder Jod steht,

gegebenenfalls in Gegenwart eines Säureakzeptors und gegebenenfalls in Gegenwart eines Verdünnungsmittels umsetzt.

Die als Ausgangsstoffe zu verwendenden Verbindungen sind durch die Formeln (II) und (III) definiert. Vorzugsweise stehen in diesen Formeln R, $R^1$, $R^2$ und $R^3$ sowie n für diejenigen Reste bzw. Symbole, welche bereits im Rahmen der Definition der Formel (I) als bevorzugt genannt sind.

Ausgangsverbindungen der Formeln (II) und (III) sind bekannt oder lassen sich noch im Prinzip bekannten Verfahren herstellen (vgl. EP-OS 5 501).

Das Verfahren zur Herstellung der erfindungsgemäß verwendbaren Oxycarbonsäureamide der Formel (I) wird vorzugsweise unter Verwendung geeigneter Lösungs- oder Verdünnungsmittel durchgeführt. Als solche kommen praktisch alle inerten organischen Solventien in Frage. Hierzu gehören insbesondere Alkohole, wie Methanol, Äthanol, n- und iso-Propanol, n-, iso-, sek.- und tert.-Butanol, Äther wie Diethyläther, Dibutyläther, Tetrahydrofuran und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können bei dem Verfahren praktisch alle üblicherweise verwendbaren Säurebindemittel eingesetzt werden: hierzu gehören insbesondere Alkali- und Erdalkalihydroxide bzw. -oxide wie Natrium- und Kaliumhydroxid sowie Calciumoxid oder Calcium-hydroxid, Alkali- und Erdalkali-carbonate wie Natrium-, Kalium- und Calciumcarbonat, Alkalialkoholate wie Natrium-methylat, -ethylat und -tert.-butylat, Kaliummethylat, -ethylat und -tert.-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann innerhalb eines größeren Bereichs variiert werden. Im allgemeinen arbeitet man

zwischen -50 und +150°C, vorzugsweise zwischen -20 und +100°C.

Das Verfahren wird im allgemeinen bei Normaldruck durchgeführt.

Bei der Durchführung des Verfahrens zur Herstellung der erfindungsgemäß verwendbaren Oxycarbonsäureamide setzt man auf 1 Mol Halogenazol der Formel (III) 1,0 bis 1,5 Mol α-Hydroxy-carbonsäureamid der Formel (II) ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden: man destilliert gegebenenfalls einen Teil des Verdünnungsmittels unter vermindertem Druck ab und gießt den Rest der Reaktionsmischung in Wasser. Soweit die Produkte hierbei kristallin anfallen, werden sie durch Absaugen isoliert. Andernfalls werden die organischen Produkte mit einem mit Wasser nicht mischbaren Lösungsmittel wie z.B. Toluol oder Methylenchlorid extrahiert; nach Waschen und Trocknen wird dann von der organischen Phase das Lösungsmittel im Vakuum abdestilliert. Die zurückbleibenden Produkte werden durch ihren Schmelzpunkt bzw. ihren Brechungsindex charakterisiert.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak,



BAD ORIGINAL

Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Einige der erfindungsgemäß verwendbaren Oxycarbonsäureamide der Formel (I) üben auch eine synergistische Wirksamkeit auf Insektizide aus.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie

Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen o,l und 95 Gewichtsprozent Wirkstoff, vorzugsweise zwischen o,5 und 9o %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche o,ol bis 5o kg, bevorzugt o,o5 bis lo kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die gute pflanzenwachstumsregulierende Wirksamkeit und die Herstellung der erindungsgemäß verwendbaren Oxycarbonsäureamide der Formel (I) gehen aus den nachfolgenden Beispielen hervor.

In den Verwendungsbeispielen werden die nachstehend aufgeführten Stoffe als Vergleichssubstanzen eingesetzt:

$Cl - CH_2 - CH_2 - \overset{\oplus}{N}(CH_3)_3 \quad Cl^{\ominus}$ = (A)

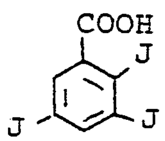

= (B)

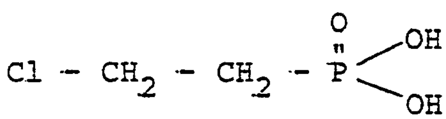

= (C)

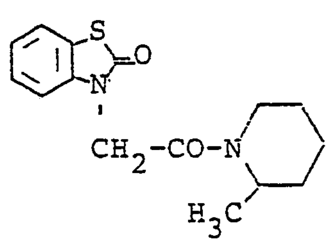

= (D)

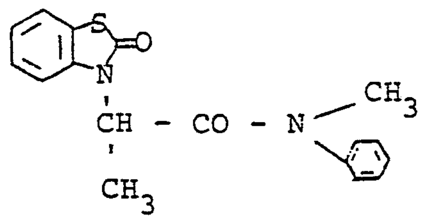

= (E)

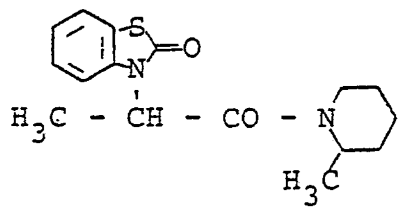

= (F)

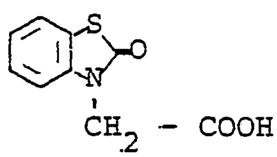

= (G)

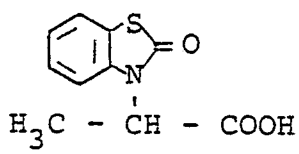

= (H)

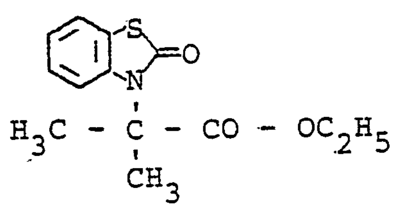

= (J)

Off-Shoot-T ® = (K)

(Wachstumsregulator auf Basis von Fettalkoholen mit 6, 8, 10 und 12 Kohlenstoffatomen).

Beispiel A

Wuchshemmung bei Sojabohnen

Lösungsmittel : 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Wirkstoffkonzentrationen und Versuchsergebnisse gehen aus der nachfolgenden Tabelle hervor.

Tabelle A

Wuchshemmung bei Sojabohnen

| Wirkstoff | Wirkstoffkonzentration in % | Wuchshemmung in % |
|---|---|---|
| - (Kontrolle) | 0 | 0 |
| (A) (bekannt) | 0,05 | 45 |
| (D) (bekannt) | 0,05 | 0 |
| (E) (bekannt) | 0,05 | 0 |
| (F) (bekannt) | 0,05 | 0 |
| (H) (bekannt) | 0,05 | 35 |
| (16) | 0,05 | 60 |
| (17) | 0,05 | 60 |
| (18) | 0,05 | 90 *) |
| (19) | 0,05 | 70 |
| (20) | 0,05 | 100 |

*) vermehrte Seitentriebbildung

Tabelle A (Fortsetzung)

Wuchshemmung bei Sojabohnen

| Wirkstoff | Wirkstoffkonzentration in % | Wuchshemmung in % |
|---|---|---|
| (44) | 0,05 | 90**) |
| (45) | 0,05 | 50 |
| (46) | 0,05 | 50 |
| (47) | 0,05 | 60 |
| (48) | 0,05 | 75**) |
| (49) | 0,05 | 55 |
| (50) | 0,05 | 55 |
| (85) | 0,05 | 50 |
| (86) | 0,05 | 95 |
| (91) | 0,05 | 100**) |
| (94) | 0,05 | 70 |

**) dunkelgrüne Blattfarbe

Beispiel B

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitung besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Wirkstoffkonzentrationen und Versuchsergebnisse gehen aus der nachfolgenden Tabelle hervor.

Tabelle B

Wuchshemmung bei Baumwolle

| Wirkstoff | Wirkstoffkonzentration in % | Wuchshemmung in % |
| --- | --- | --- |
| - (Kontrolle) | 0 | 0 |
| (B) (bekannt) | 0,05 | 25 |
| (D) (bekannt) | 0,05 | 0 |
| (E) (bekannt) | 0,05 | 0 |
| (F) (bekannt) | 0,05 | 0 |
| (H) (bekannt) | 0,05 | 0 |
| (J) (bekannt) | 0,05 | 0 |
| (4) | 0,05 | 70 |
| (6) | 0,05 | 100 |
| (7) | 0,05 | 45 |
| (8) | 0,05 | 60 |
| (9) | 0,05 | 100 |
| (10) | 0,05 | 85 |

Tabelle B (Fortsetzung)

Wuchshemmung bei Baumwolle

| Wirkstoff | Wirkstoffkonzentration in % | Wuchshemmung in % |
|---|---|---|
| (11) | 0,05 | 60 |
| (12) | 0,05 | 55 |
| (13) | 0,05 | 55 |
| (14) | 0,05 | 45 |
| (15) | 0,05 | 45 |
| (16) | 0,05 | 45 |
| (18) | 0,05 | 40 |
| (19) | 0,05 | 45 |
| (20) | 0,05 | 90 |
| (21) | 0,05 | 65 |
| (22) | 0,05 | 65 |
| (23) | 0,05 | 55 |
| (24) | 0,05 | 90 |
| (25) | 0,05 | 85 |
| (26) | 0,05 | 80 |
| (27) | 0,05 | 100 |
| (46) | 0,05 | 35 |
| (48) | 0,05 | 30 |

Tabelle B (Fortsetzung)

Wuchshemmung bei Baumwolle

| Wirkstoff | Wirkstoffkonzentration in % | Wuchshemmung in % |
|---|---|---|
| (49) | 0,05 | 60 |
| (50) | 0,05 | 30 |
| (51) | 0,05 | 30 |
| (52) | 0,05 | 60 |
| (63) | 0,05 | 65 |
| (64) | 0,05 | 40 |
| (65) | 0,05 | 50 |
| (66) | 0,05 | 85 |
| (67) | 0,05 | 65 |
| (68) | 0,05 | 85 |
| (76) | 0,05 | 35 |
| (77) | 0,05 | 35 |
| (78) | 0,05 | 35 |
| (33) | 0,05 | 65 |
| (81) | 0,05 | 55 |
| (82) | 0,05 | 50 |
| (87) | 0,05 | 95 |
| (88) | 0,05 | 55 *) |

*) Seitentriebe im Wuchs gehemmt

Tabelle B (Fortsetzung)

Wuchshemmung bei Baumwolle

| Wirkstoff | Wirkstoffkonzentration in % | Wuchshemmung in % |
|---|---|---|
| (89) | 0,05 | 35*) |
| (90) | 0,05 | 55*) |
| (91) | 0,05 | 100**) |
| (92) | 0,05 | 65 |
| (93) | 0,05 | 100 |
| (94) | 0,05 | 55 |

*) Seitentriebe im Wuchs gehemmt

**) dunkelgrüne Blätter

Beispiel C

Wuchshemmung bei Gras (Festuca pratensis)

Lösungsmittel : 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Wirkstoffkonzentrationen und Versuchsergebnisse gehen aus der nachfolgenden Tabelle hervor.

Tabelle C

Wuchshemmung bei Gras (Festuca pratensis)

| Wirkstoff | Wirkstoffkonzentration in % | Wuchshemmung in % |
|---|---|---|
| - (Kontrolle) | 0 | 0 |
| (C) (bekannt) | 0,05 | 15 |
| (D) (bekannt) | 0,05 | 0 |
| (E) (bekannt) | 0,05 | 0 |
| (F) (bekannt) | 0,05 | 0 |
| (G) (bekannt) | 0,05 | 0 |
| (H) (bekannt) | 0,05 | 0 |
| (J) (bekannt) | 0,05 | 10 |
| (1) | 0,05 | 60 |
| (2) | 0,05<br>0,025<br>0,0125 | 100*)<br>72<br>18 |

*) dunkelgrüne Färbung

Tabelle C (Fortsetzung)

Wuchshemmung bei Gras (Festuca pratensis)

| Wirkstoff | Wirkstoffkonzentration in % | Wuchshemmung in % |
|---|---|---|
| ( 5) | 0,05 | 95 |
| ( 6) | 0,05 | 100 |
| ( 7) | 0,05 | 95 |
| ( 8) | 0,05 | 95 |
| ( 9) | 0,05 | 95 |
| (10) | 0,05 | 100 |
| (12) | 0,05 | 100*) |
| (13) | 0,05 | 100*) |
| (14) | 0,05 | 95 |
| (16) | 0,05 | 100 |
| (17) | 0,05 | 74*) |
| | 0,025 | 53*) |
| | 0,0125 | 19 |
| (18) | 0,05 | 90*) |
| (19) | 0,05 | 100 |
| (20) | 0,05 | 95 |
| (21) | 0,05 | 90*) |
| (22) | 0,05 | 97 |
| | 0,025 | 30 |

*) dunkelgrüne Färbung

Tabelle C (Fortsetzung)

Wuchshemmung bei Gras (Festuca pratensis)

| Wirkstoff | Wirkstoffkonzentration in % | Wuchshemmung in % |
|---|---|---|
| (23) | 0,05 | 70 |
| (24) | 0,05 | 95 |
| (26) | 0,05 | 100 |
| (27) | 0,05 | 95 |
| (28) | 0,05 | 45 |
| (29) | 0,05 | 90 *) |
| (30) | 0,05 | 100 |
| (31) | 0,05 | 100 |
| (32) | 0,05 | 45 |
| (33) | 0,05 | 100 |
| (44) | 0,05 | 90 |
| (45) | 0,05 | 100 |
| (46) | 0,05 | 100 |
| (47) | 0,05 | 100 |
| (48) | 0,05 | 100 |
| (49) | 0,05 | 100 |
| (52) | 0,05 | 100 |
| (53) | 0,05 | 100 |
| (54) | 0,05 | 55 |
| (55) | 0,05 | 70 |

*) dunkelgrüne Färbung

Tabelle C (Fortsetzung)

Wuchshemmung bei Gras (Festuca pratensis)

| Wirkstoff | Wirkstoffkonzentration in % | Wuchshemmung in % |
|---|---|---|
| (56) | 0,05 | 65 |
| (62) | 0,05 | 100 |
| (63) | 0,05 | 100 |
| (64) | 0,05 | 75 |
| (65) | 0,05 | 85 |
| (66) | 0,05 | 95 |
| (67) | 0,05 | 95 |
| (68) | 0,05 | 95 |
| (69) | 0,05 | 100 |
| (70) | 0,05 | 100 |
| (71) | 0,05 | 100 |
| (72) | 0,05 | 100 |
| (73) | 0,05 | 100 |
| (74) | 0,05 | 100 |
| (75) | 0,05 | 100 |
| (76) | 0,05 | 100 |
| (77) | 0,05 | 100 |
| (78) | 0,05 | 100 |

Tabelle C (Fortsetzung)

Wuchshemmung bei Gras (Festuca pratensis)

| Wirkstoff | Wirkstoffkonzentration in % | Wuchshemmung in % |
|---|---|---|
| (79) | 0,05 | 85 |
| (80) | 0,05 | 70 |
| (33) | 0,05 | 60 |
| (83) | 0,05 | 60 |
| (84) | 0,05 | 60 |
| (85) | 0,05 | 85 |
| (86) | 0,05 | 80 |
| (87) | 0,05 | 95 |
| (90) | 0,05 | 75 |
| (91) | 0,05 | 85 |
| (92) | 0,05 | 100 |
| (94) | 0,05 | 50 |

Beispiel D

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Wirkstoffkonzentrationen und Versuchsergebnisse gehen aus der nachfolgenden Tabelle hervor.

Tabelle D

Wuchshemmung bei Gerste

| Wirkstoff | Wirkstoffkonzentration in % | Wuchshemmung in % |
|---|---|---|
| - (Kontrolle) | 0 | 0 |
| (A) (bekannt) | 0,05 | 20 |
| (D) (bekannt) | 0,05 | 5 |
| (E) (bekannt) | 0,05 | 5 |
| (F) (bekannt) | 0,05 | 0 |
| (G) (bekannt) | 0,05 | 0 |
| (H) (bekannt) | 0,05 | 10 |
| (J) (bekannt) | 0,05 | 0 |
| (1)<br>(4) | 0,05<br>0,05 | 70*)<br>75*) |
| (5)<br>(6) | 0,05<br>0,05 | 45<br>90 |

*) starke Bestockung

Tabelle D (Fortsetzung)

Wuchshemmung bei Gerste

| Wirkstoff | Wirkstoffkonzentration in % | Wuchshemmung in % |
|---|---|---|
| ( 7) | 0,05 | 95 |
| (12) | 0,05 | 65 |
| (13) | 0,05 | 40 |
| (15) | 0,05 | 65 |
| (16) | 0,05 | 36*)**) |
| (17) | 0,05 | 45 |
| (18) | 0,05 | 40 |
| (19) | 0,05 | 65 |
| (20) | 0,05 | 55 |
| (26) | 0,05 | 90 |
| (27) | 0,05 | 40 |
| (28) | 0,05 | 35 |
| (30) | 0,05 | 65 |
| (31) | 0,05 | 90 |
| (44) | 0,05 | 90 |
| (45) | 0,05 | 50*) |
| (46) | 0,05 | 95*) |
| (47) | 0,05 | 95*) |

*) starke Bestockung

**) dunkelgrüne Blattfärbung

Tabelle D (Fortsetzung)

Wuchshemmung bei Gerste

| Wirkstoff | Wirkstoffkonzentration in % | Wuchshemmung in % |
|---|---|---|
| (48) | 0,05 | 95*) |
| (49) | 0,05 | 0*) |
| (50) | 0,05 | 75*) |
| (51) | 0,05 | 75*) |
| (52) | 0,05 | 60*) |
| (53) | 0,05 | 95*) |
| (57) | 0,05 | 80*) |
| (58) | 0,05 | 80*) |
| (75) | 0,05 | 75 |
| (84) | 0,05 | 30 |
| (86) | 0,05 | 55 |
| (94) | 0,05 | 30 |

*) starke Bestockung

Beispiel E

Wuchsbeeinflussung bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Wirkstoffe, Wirkstoffkonzentrationen und Versuchsergebnisse gehen aus der nachfolgenden Tabelle hervor.

Tabelle E

Wuchsbeeinflussung bei Zuckerrüben

| Wirkstoff | Wirkstoffkonzentration in % | Wuchsbeeinflussung in % |
|---|---|---|
| - (Kontrolle) | 0 | 0 |
| (A) (bekannt) | 0,05 | -10 |
| (1) | 0,05 | -45 |
| (2) | 0,05 | -40 |
| (4) | 0,05 | -85 *) |
| (6) | 0,05 | -65 |
| (7) | 0,05 | -65 *) |
| (8) | 0,05 | -70 |
| (10) | 0,05 | -35 |
| (11) | 0,05 | -60 |
| (15) | 0,05 | -70 *) |
| (16) | 0,05 | -40 |
| (17) | 0,05 | -45 |
| (18) | 0,05 | + 5 |
| (19) | 0,05 | -75 **) |
| (20) | 0,05 | -55 |

*) dicke Blätter

**) dunkelgrüne Blattfärbung

Tabelle E (Fortsetzung)

Wuchsbeeinflussung bei Zuckerrüben

| Wirkstoff | Wirkstoffkonzentration in % | Wuchsbeeinflussung in % |
|---|---|---|
| (24) | 0,05 | -55 |
| (26) | 0,05 | -65 |
| (27) | 0,05 | -50 |
| (28) | 0,05 | -70 |
| (29) | 0,05 | + 5 |
| (31) | 0,05 | -35 |
| (33) | 0,05 | -100**) |
| (34) | 0,05 | -90 |
| (35) | 0,05 | -60 |
| (36) | 0,05 | + 5 |
| (37) | 0,05 | + 5 |
| (38) | 0,05 | + 5 |
| (39) | 0,05 | + 5 |
| (40) | 0,05 | -45 |
| (41) | 0,05 | -35 |
| (42) | 0,05 | -60 |
| (43) | 0,05 | -35**) |
| (44) | 0,05 | -25 |

**) dunkelgrüne Blätter

Tabelle E (Fortsetzung)

Wuchsbeeinflussung bei Zuckerrüben

| Wirkstoff | Wirkstoffkonzentration in % | Wuchsbeeinflussung in % |
|---|---|---|
| (45) | 0,05 | -25 |
| (46) | 0,05 | -85 |
| (47) | 0,05 | -45 |
| (48) | 0,05 | -60 *) |
| (49) | 0,05 | -35 |
| (50) | 0,05 | -75 |
| (51) | 0,05 | -75 *) |
| (52) | 0,05 | -75 |
| (53) | 0,05 | -25 |
| (54) | 0,05 | -25 |
| (55) | 0,05 | -45 |
| (56) | 0,05 | -20 |
| (57) | 0,05 | -85 *) |
| (58) | 0,05 | -25 |
| (59) | 0,05 | -35 |
| (60) | 0,05 | -35 |
| (61) | 0,05 | -50 |
| (63) | 0,05 | -70 |

*) dicke Blätter

Tabelle E (Fortsetzung)

Wuchsbeeinflussung bei Zuckerrüben

| Wirkstoff | Wirkstoffkonzentration in % | Wuchsbeeinflussung in % |
|---|---|---|
| (64) | 0,05 | -80 |
| (65) | 0,05 | -80*) |
| (66) | 0,05 | -54*) |
| (67) | 0,05 | -70 |
| (68) | 0,05 | -20 |
| (70) | 0,05 | -25 |
| (73) | 0,05 | -54 |
| (74) | 0,05 | -65 |
| (78) | 0,05 | -20 |
| (79) | 0,05 | -25 |
| (80) | 0,05 | -45 |
| (81) | 0,05 | -90 |
| (83) | 0,05 | -90 |
| (84) | 0,05 | -65 |
| (85) | 0,05 | -80 |
| (86) | 0,05 | -80*) |
| (87) | 0,05 | -55*) |
| (89) | 0,05 | -70 |

*) dicke Blätter

Tabelle E (Fortsetzung)

Wuchsbeeinflussung bei Zuckerrüben

| Wirkstoff | Wirkstoffkonzentration in % | Wuchsbeeinflussung in % |
|---|---|---|
| (91) | 0,05 | -90 |
| (92) | 0,05 | -55 |
| (33) | 0,05 | -85 |
| (94) | 0,05 | -45 |

Beispiel F

Hemmung des Seitentriebwachstums bei Tabak

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Tabakpflanzen werden im Gewächshaus bis zur Entfaltung des 7. Folgeblattes angezogen. In diesem Stadium wird die apikale Vegetationsspitze entfernt und die Pflanzen trofpnaß mit den Wirkstoffzubereitungen besprüht. Nach 3 Wochen werden die Seitentriebe der Pflanzen herausgebrochen und gewogen. Das Gewicht der Seitentriebe der behandelten Pflanzen wird mit dem der Kontrollpflanzen verglichen. 100 % Hemmung bedeuten das Fehler von Seitentrieben und 0 % ein Wachstum entsprechend dem der Kontrollpflanzen.

Wirkstoffe, Wirkstoffkonzentrationen und Versuchsergebnisse gehen aus der nachfolgenden Tabelle hervor.

Tabelle F

Hemmung des Seitentriebwachstums bei Tabak

| Wirkstoff | Wirkstoffkonzentration in % | Wuchshemmung der Seitentriebe in % |
|---|---|---|
| - (Kontrolle) | 0 | 0 |
| (K) (bekannt) | 0,2 | 20 |
| (2) | 0,2 | 85 |
| | 0,1 | 64 |
| (16) | 0,2 | 66 |
| (19) | 0,2 | 100 |
| | 0,1 | 99 |
| (20) | 0,2 | 100*) |
| | 0,1 | 100*) |
| (21) | 0,2 | 97 |
| | 0,1 | 97 |
| (22) | 0,2 | 97 |
| | 0,1 | 74 |
| (26) | 0,2 | 52 |
| (27) | 0,2 | 99 |
| | 0,1 | 96 |

*) Auffallend große Blätter

Tabelle F (Fortsetzung)

Hemmung des Seitentriebwachstums bei Tabak

| Wirkstoff | Wirkstoffkonzentration in % | Wuchshemmung der Seitentriebe in % |
| --- | --- | --- |
| (31) | 0,2 | 87 |

Beispiel G

Entlaubung und Austrocknung der Blätter bei Baumwolle

Lösungsmittel: 30 Gewichsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall.

Wirkstoffe, Wirkstoffkonzentrationen und Versuchsergebnisse gehen aus der folgenden Tabelle hervor.

Tabelle G

Entlaubung und Austrocknung der Blätter bei Baumwolle

| Wirkstoff | Wirkstoffkonzentration in % | Wirkung |
|---|---|---|
| - (Kontrolle) | 0 | 0 |
| (25) | 0,05 | +++ |

Herstellungsbeispiele

Beispiel 1:

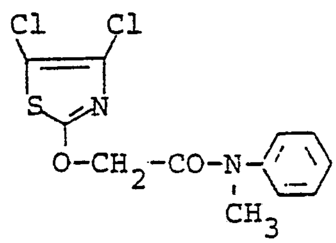

14,8 g (0,09 Mol) Hydroxyessigsäure-N-methylanilid werden in 100 ml Isopropanol gelöst. Die Lösung wird mit 5,6 g gepulvertem Kaliumhydroxyd verrührt und auf -15°C gekühlt. Bei -15°C werden in einer Stunde 13,1 g (0,07 Mol) 2,4,5-Trichlorthiazol hinzugetropft. Man läßt 3 Stunden bei -10°C rühren und anschließend die Temperatur auf 0°C ansteigen, rührt 2 Stunden bei 0°C und anschließend 10 Stunden bei Raumtemperatur. Dann werden 3/4 des Lösungsmittels abdestilliert, der Rückstand wird auf Wasser gegossen und der ausfallende Festkörper abgesaugt und getrocknet. Die Ausbeute beträgt 18 g (81 % der Theorie); Schmelzpunkt: 82°C;

0-(4,5-Dichlor-1,3-thiazol-2-yl)-oxyessigsäure-N-methylanilid.

Beispiel 2:

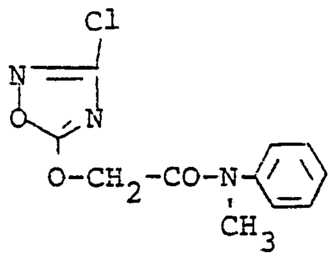

9 g (0,05 Mol) Hydroxyessigsäure-N-methylanilid werden in 100 ml Acetonitril gelöst. Dann werden 7,6 g Kaliumcarbonat hinzugegeben. Bei -10°C werden 6,9 g 3,5-Dichlor-1,2,4-oxadiazol langsam zugetropft. Dann wird noch 2 Stunden bei -10°C, 3 Stunden bei 0 - 5°C und 20 Stunden bei Raumtemperatur nachgerührt. Dann wird das Reaktionsgemisch auf Wasser gegossen und das Produkt mit Toluol extrahiert. Nach dem Abdestillieren des Toluols kristallisiert die Substanz. Die Ausbeute beträgt 9 g ( 70 % der Theorie); Schmelzpunkt 73°C; 0-(3-Chlor-1,2,4-oxadiazol-5-yl)-oxyessigsäure-N-methylanilid.

Beispiel 3:

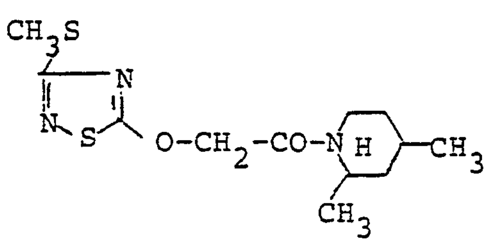

8,3 g (0,05 Mol) 5-Chlor-3-methylthio-1,2,4-thiadiazol werden bei 20 - 30°C zu einer Mischung aus 8,5 g (0,05 Mol) Hydroxyessigsäure-2,4-dimethylpiperidid, 3,4 g (0,06 Mol) Kaliumhydroxid-Pulver, 1 g Kupferpulver und 100 ml iso-Propanol gegeben. Die Mischung wird mehrere Stunden gerührt und anschließend mit Wasser verdünnt, dann extrahiert man das Produkt mit Toluol, wäscht die organische Phase mit Wasser, trocknet, filtriert und zieht vom Filtrat das Lösungsmittel im Vakuum ab.

Die Ausbeute beträgt 9 g (60 % der Theorie); Brechungsindex: $n_D^{23}$ = 1,5460;

0-(3-Methylthio-1,2,4-thiadiazol-5-yl)-oxyessigsäure-N-(2,4-dimethylpiperidid).

Beispiel 4:

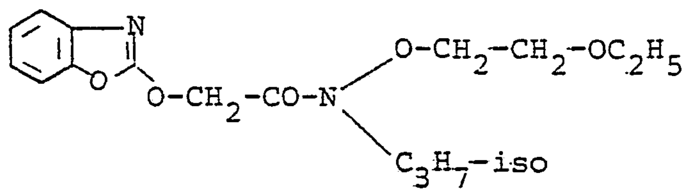

Zu einer Lösung von 6,2 g (0,11 Mol) Kaliumhydroxid in 200 ml Isopropanol tropft man bei 0 - 5°C erst 20,5 g (0,1 Mol) α-Hydroxy-N-iso-propyl-acethydroxamsäure-0-(2-ethoxyethyl)-ester und dann 15,4 g (0,1 Mol) 2-Chlorbenzoxazol zu und rührt das Gemisch anschließend

6 Stunden ohne Kühlung nach. Dann destilliert man das Lösungsmittel im Vakuum ab, löst den Rückstand in 200 ml Toluol und schüttelt einmal mit 100 ml Wasser aus. Die organische Phase wird über Natriumsulfat getrocknet und im Vakuum eingedampft. Der Rückstand wird bei 60°C im Hochvakuum andestilliert. Man erhält auf diese Weise 30,3 g (94 % der Theorie) 2-Benzoxazol-2-yl-oxy-N-isopropyl-acethydroxamsäure-0-(2-ethoxyethyl)-ester in Form eines hellbraunen Öles mit dem Brechungsindex $n_D^{22}$ = 1,4986.

Nach den in den Beispielen 1 bis 4 beschriebenen Methoden werden auch die in den nachfolgenden Beispielen formelmäßig aufgeführten Oxycarbonsäureamide der Formel (I) hergestellt.

| Beispiel Nr. | Strukturformel | Schmelzpunkt (°C) bzw. Brechungsindex |
|---|---|---|
| 5 | 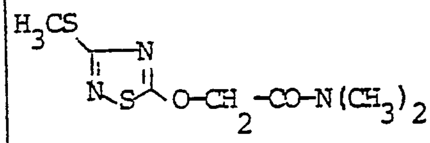 | 150 |
| 6 | 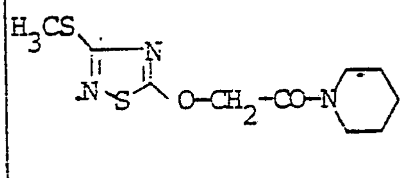 | $n_D^{23}=1,544$ |
| 7 | 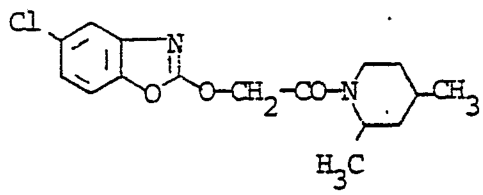 | |
| 8 | 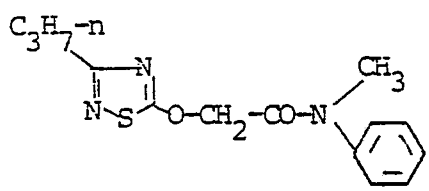 | 47 |
| 9 | 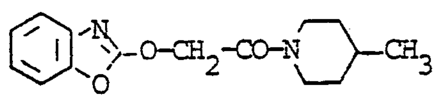 | |
| 10 | 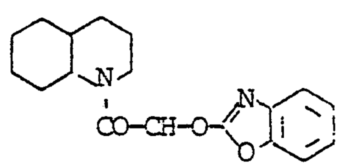 | |

| Beispiel Nr. | Strukturformel | Schmelzpunkt (°C) bzw. Brechungsindex |
| --- | --- | --- |
| 11 | 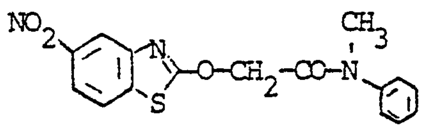 | |
| 12 | 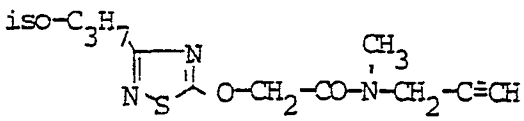 | $n_D^{21}$=1,5230 |
| 13 | 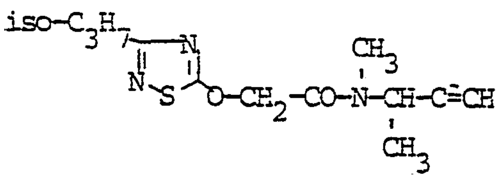 | $n_D^{21}$=1,5155 |
| 14 | 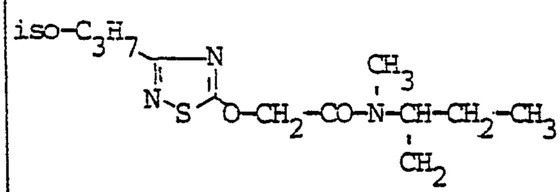 | 46 - 48 |
| 15 | 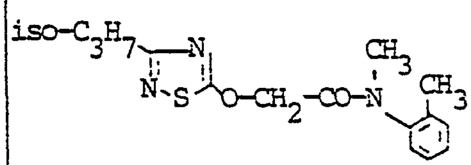 | $n_D^{21}$=1,5428 |
| 16 | 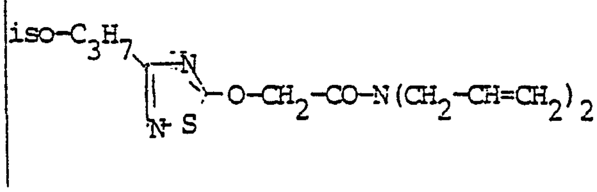 | $n_D^{23}$=1,5142 |

| Beispiel Nr. | Strukturformel | Schmelzpunkt (°C) bzw. Brechungsindex |
|---|---|---|
| 17 | 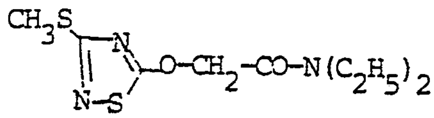 | 68 |
| 18 | 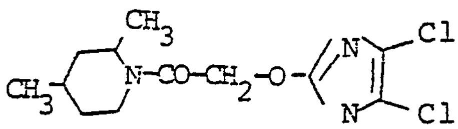 | $n_D^{22}$=1,5432 |
| 19 | 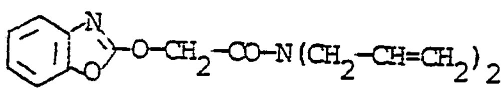 | |
| 20 | 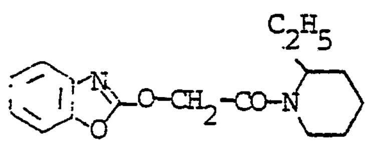 | |
| 21 | 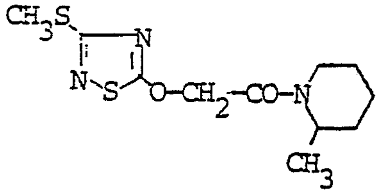 | $n_D^{23}$=1,5560 |
| 22 | 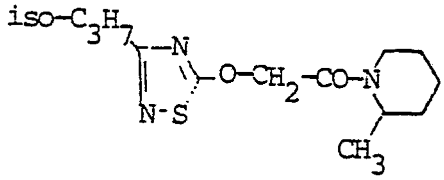 | $n_D^{23}$=1,5135 |

| Beispiel Nr. | Strukturformel | Schmelzpunkt (°C) bzw. Brechungsindex |
|---|---|---|
| 23 | 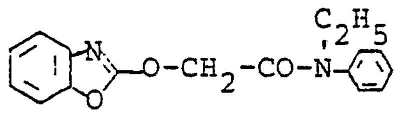 | |
| 24 | 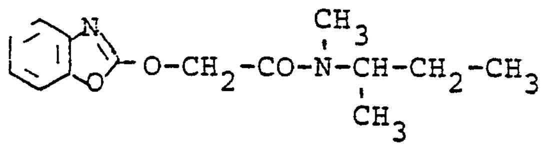 | |
| 25 | 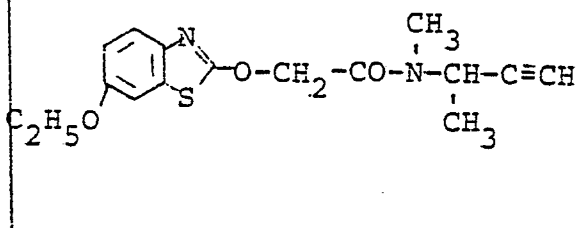 | |
| 26 | 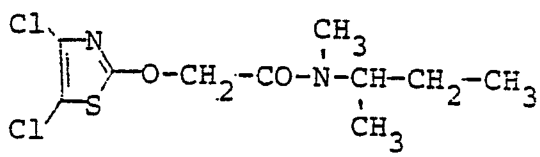 | $n_D^{25}=1,5357$ |
| 27 | 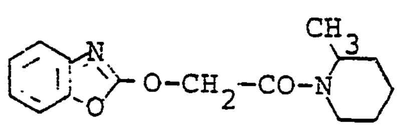 | |
| 28 | 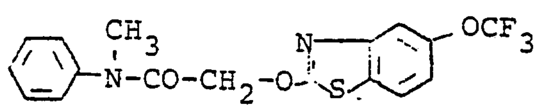 | |

| Beispiel Nr. | Strukturformel | Schmelzpunkt (°C) bzw. Brechungsindex |
|---|---|---|
| 29 | 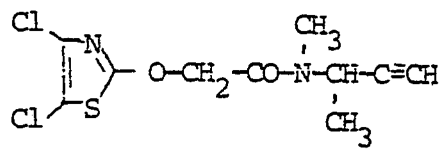 | |
| 30 | 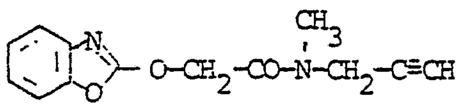 | |
| 31 | 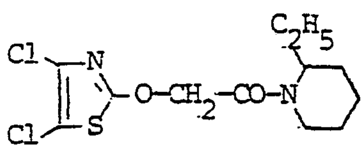 | $n_D^{21}$=1,5440 |
| 32 | 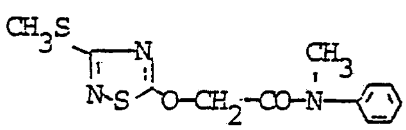 | 98 |
| 33 | 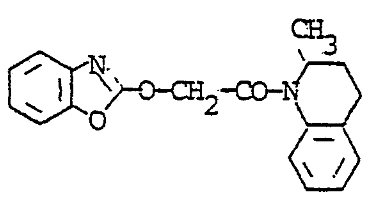 | |
| 34 | 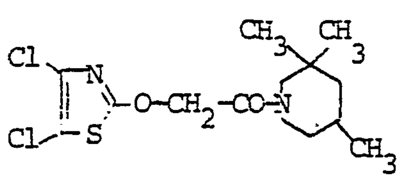 | 90 |

| Beispiel Nr. | Strukturformel | Schmelzpunkt (°C) bzw. Brechungsindex |
|---|---|---|
| 35 | 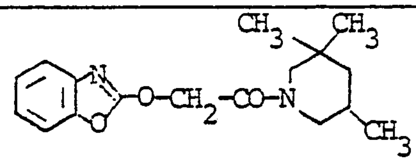 | |
| 36 | 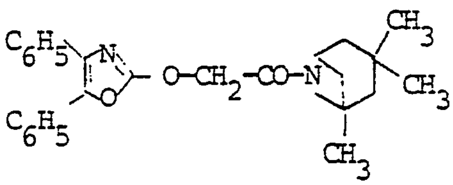 | 133 |
| 37 | 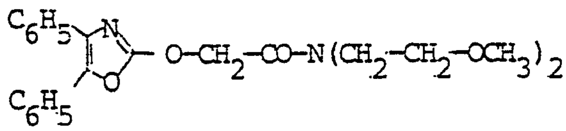 | 92 |
| 38 | 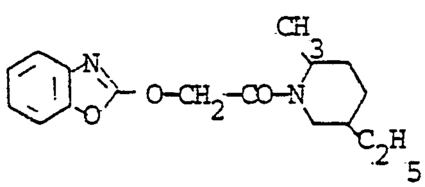 | |
| 39 | 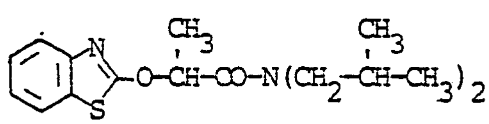 | |
| 40 | 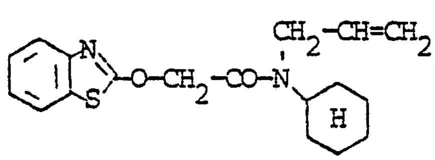 | |

| Beispiel Nr. | Strukturformel | Schmelzpunkt (°C) bzw. Brechungsindex |
|---|---|---|
| 41 | 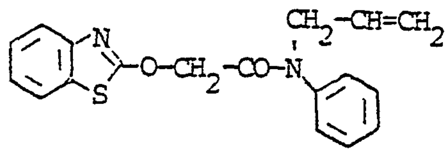 | |
| 42 | 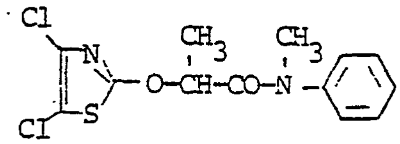 | $n_D^{22}$=1,5584 |
| 43 | 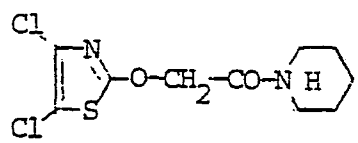 | 62 |
| 44 | 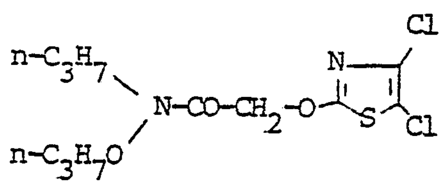 | |
| 45 | 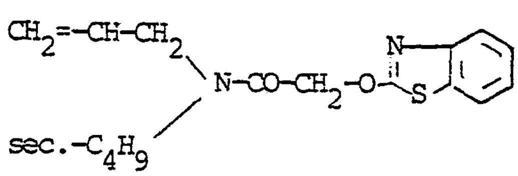 | |
| 46 | 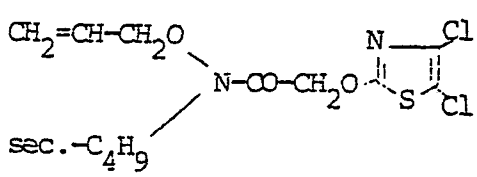 | |

| Beispiel Nr. | Strukturformel | Schmelzpunkt (°C) bzw. Brechungsindex |
|---|---|---|
| 47 | 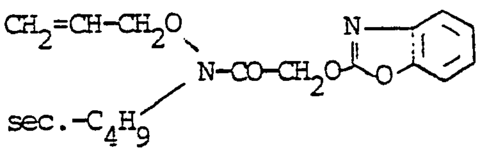 | |
| 48 | 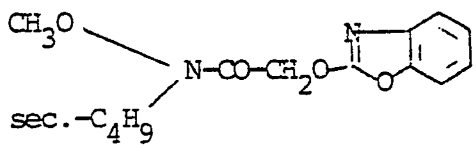 | |
| 49 | 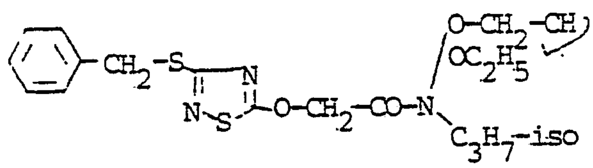 | |
| 50 | 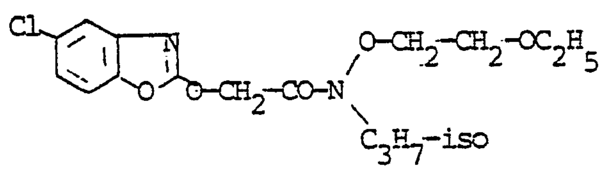 | |
| 51 | 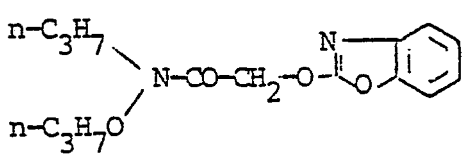 | |
| 52 | 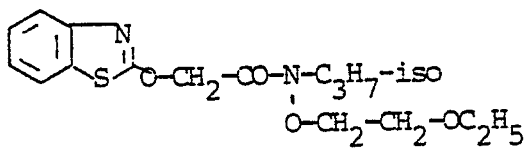 | |

| Beispiel Nr. | Strukturformel | Schmelzpunkt (°C) bzw. Brechungsindex |
| --- | --- | --- |
| 53 | 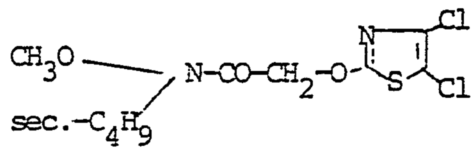 | |
| 54 | 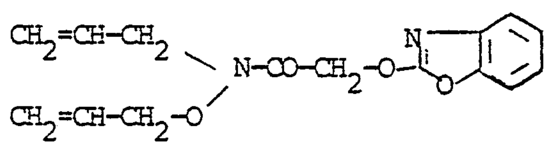 | |
| 55 | 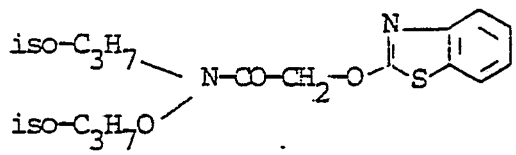 | |
| 56 | 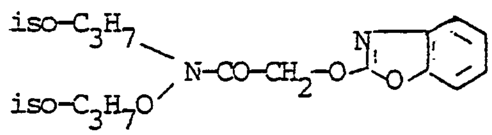 | |
| 57 | 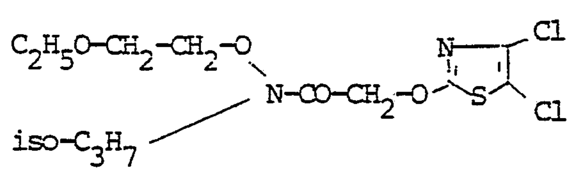 | |
| 58 | 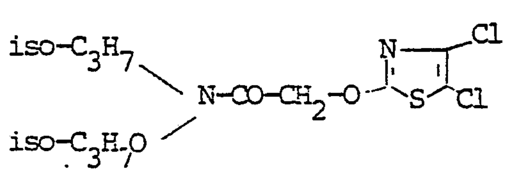 | |

| Beispiel Nr. | Strukturformel | Schmelzpunkt (°C) bzw. Brechungsindex |
|---|---|---|
| 59 | 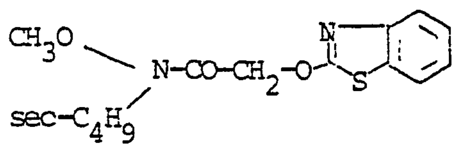 | |
| 60 | 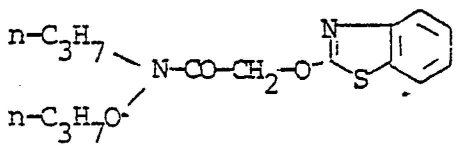 | |
| 61 | 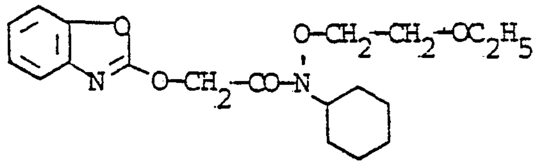 | |
| 62 | 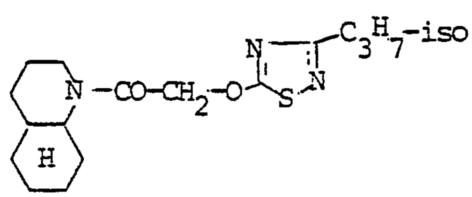 | $n_D^{23}=1,5291$ |
| 63 | 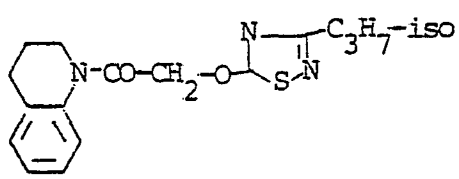 | $n_D^{23}=1,5760$ |
| 64 | 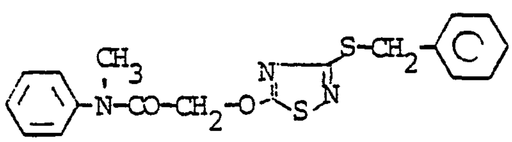 | 108 |

| Beispiel Nr. | Strukturformel | Schmelzpunkt (°C) bzw. Brechungsindex |
|---|---|---|
| 65 | 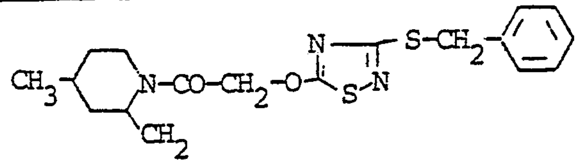 | $n_D^{19}$=1,5816 |
| 66 | 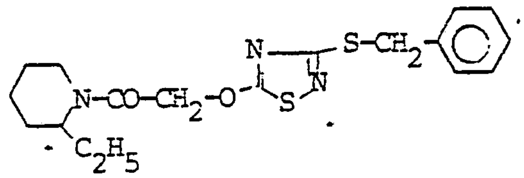 | $n_D^{19}$=1,5805 |
| 67 | 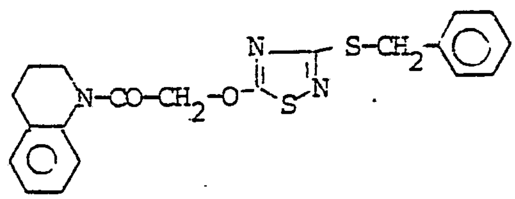 | $n_D^{19}$=1,6247 |
| 68 | 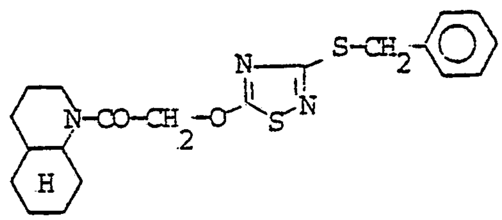 | $n_D^{19}$=1,5895 |
| 69 | 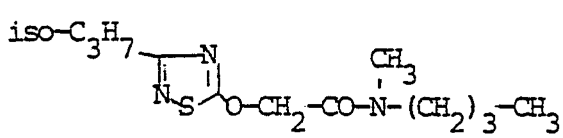 | $n_D^{20}$=1,4947 |
| 70 | 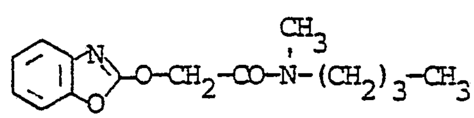 | |

| Beispiel Nr. | Strukturformel | Schmelzpunkt (°C) bzw. Brechungsindex |
|---|---|---|
| 71 | 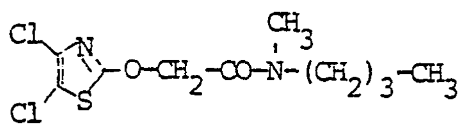 | 47 |
| 72 | 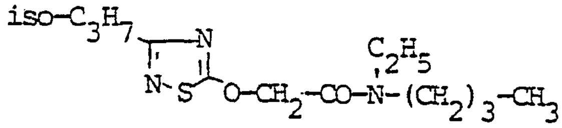 | |
| 73 | 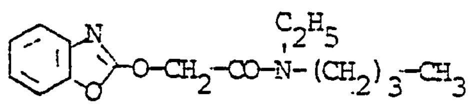 | |
| 74 | 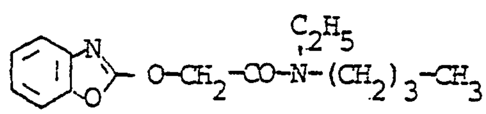 | |
| 75 | 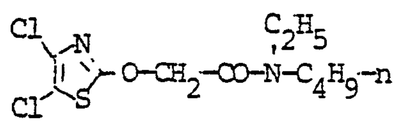 | |
| 76 | 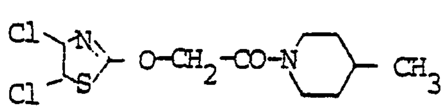 | $n_D^{20}$ =1,5505 |

| Beispiel Nr. | Strukturformel | Schmelzpunkt (°C) bzw. Brechungsindex |
|---|---|---|
| 77 | 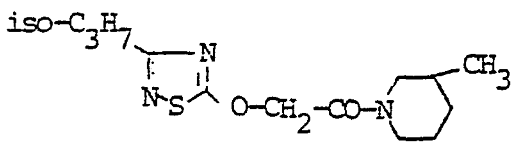 | 45 |
| 78 | 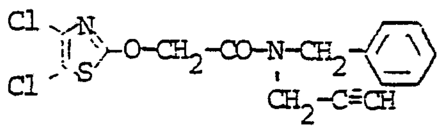 | |
| 79 | 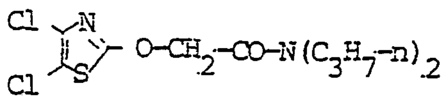 | $n_D^{22}=1,5579$ |
| 80 | 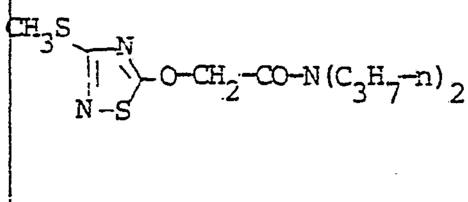 | $n_D^{21}=1,5309$ |
| 81 | 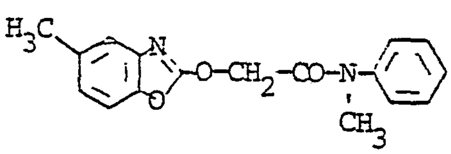 | |
| 82 | 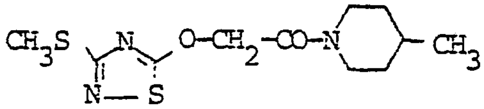 | 73 |

| Beispiel Nr. | Strukturformel | Schmelzpunkt (°C) bzw. Brechungsindex |
|---|---|---|
| 83 | 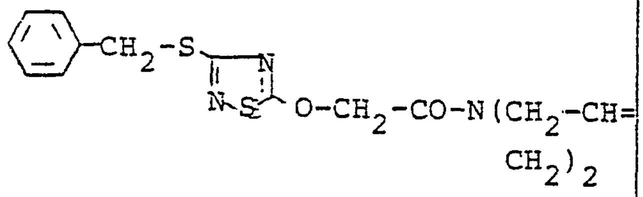 | 54 |
| 84 | 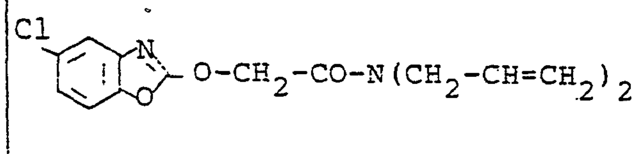 | |
| 85 | 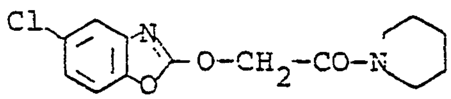 | |
| 86 | 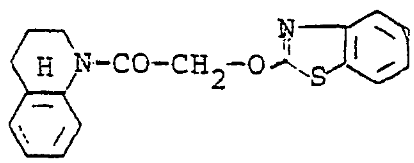 | |
| 87 | 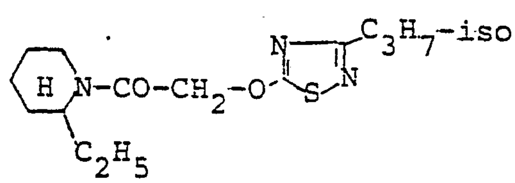 | 67 |
| 88 | 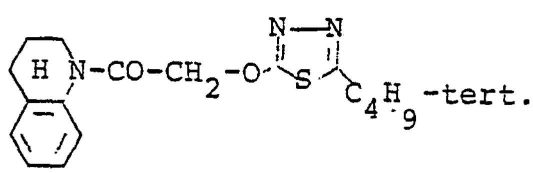 | $n_D^{23}$=1,5713 |

| Beispiel Nr. | Strukturformel | Schmelzpunkt (°C) bzw. Brechungsindex |
|---|---|---|
| 89 | 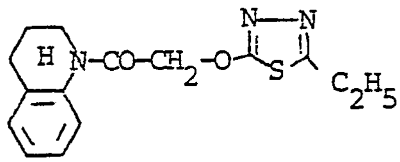 | $n_D^{23}$=1,5865 |
| 90 | 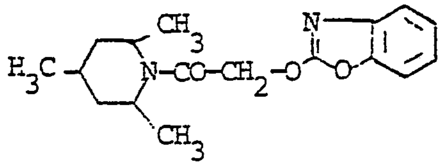 | |
| 91 | 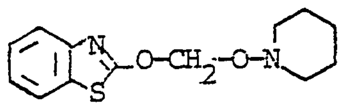 | |
| 92 | 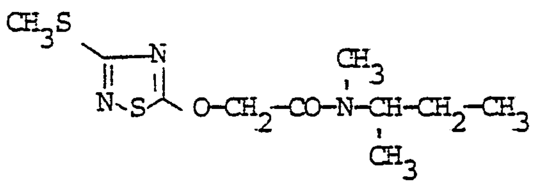 | $n_D^{20}$=1,5410 |
| 93 | 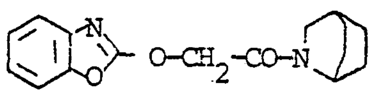 | |
| 94 | 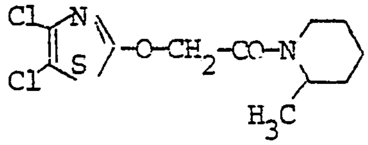 | |

Patentansprüche

1) Mittel zur Regulierung des Pflanzenwachstums, gekennzeichnet durch einen Gehalt an mindestens einem Oxycarbonsäureamid der Formel

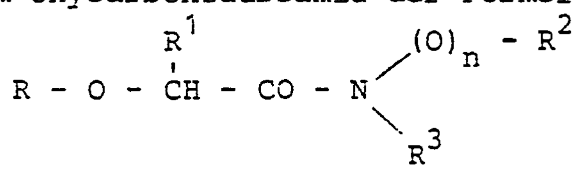

(I)

in welcher

R für einen fünfgliedrigen Heterocyclus steht, welcher ein Sauerstoff- oder ein Schwefelatom und zusätzlich 1 bis 3 Stickstoffatome enthält und welcher gegebenenfalls substituiert ist durch Halogen, Cyano, Nitro, Amino, Alkylamino, Arylamino, Dialkylamino, Alkylcarbonylamino, Alkylcarbonyl, Carboxy, Alkoxycarbonyl, Carbamoyl, Alkylaminocarbonyl, Dialkylaminocarbonyl, gegebenenfalls durch Halogen, Nitro oder Alkyl substituiertes Arylaminocarbonyl, gegebenenfalls durch Halogen, Cyano, Nitro, Alkyl oder Alkoxy substituiertes Aryl, gegebenenfalls durch Halogen substituiertes Aralkyl, gegebenenfalls durch Halogen substituiertes Alkoxy, Alkenoxy, Alkinoxy, Alkoxycarbonylalkoxy, Aralkoxy oder Aryloxy, gegebenenfalls durch Halogen substituiertes Alkylthio, Alkenylthio, Alkinylthio, Alkoxy=carbonylalkylthio, Aralkylthio oder Arylthio, gegebenenfalls durch Halogen substituiertes Alkylsulfinyl

oder Alkylsulfonyl, gegebenenfalls durch Halogen substituiertes Alkyl, Alkenyl, Alkinyl, Alkoxyalkyl, Aralkoxyalkyl, Aryloxyalkyl, Alkylthioalkyl, Alkylsulfinylalkyl, Alkylsulfonylalkyl, Arylthioalkyl, Arylsulfinylalkyl, Arylsulfonylalkyl, Carboxyalkyl, Alkoxycarbonylalkyl, gegebenenfalls substituiertes Aminocarbonylalkyl, Cyanoalkyl oder Cycloalkyl, oder welcher gegebenenfalls benzanneliert ist, wobei der Benzo-Rest gegebenenfalls durch Halogen, Alkyl, Halogenalkyl, Alkoxy, Halogenalkoxy, Alkylthio, Halogenalkylthio, Amino, Alkylamino, Dialkylamino, Nitro, Cyano, Alkoxycarbonyl oder durch gegebenenfalls halogen-substituiertes Alkylendioxy substituiert ist,

in welcher weiter

$R^1$ für Wasserstoff oder Alkyl steht,

n für Null oder 1 steht und

$R^2$ und $R^3$, welche gleich oder verschieden sein können, einzeln für Wasserstoff oder für gegebenenfalls substituierte Reste aus der Reihe Alkyl, Alkenyl, Alkinyl, Cycloalkyl, Cycloalkenyl, Aralkyl oder Aryl stehen oder, - für den Fall, daß n für Null steht -, zusammen mit dem Stickstoffatom an das sie gebunden sine, einen gegebenenfalls substituierten, gegebenenfalls

teilweise ungesättigten und gegebenenfalls benzannelierten Mono- oder Bicyclus bilden, der gegebenenfalls weitere Heteroatome enthält.

2) Verfahren zur Regulierung des Pflanzenwachstums, dadurch gekennzeichnet, daß man Oxycarbonsäureamide der Formel (I) auf die Pflanzen oder ihren Lebensraum einwirken läßt.

3) Verwendung von Oxycarbonsäureamiden der Formel (I) zur Regulierung des Pflanzenwachstums.

4) Verfahren zur Herstellung von pflanzenwachstumsregulierenden Mittel, dadurch gekennzeichnet, daß man Oxycarbonsäureamide der Formel (I) mit Streckmitteln und/oder oberflächenaktiven Stoffen vermischt.

5) Mittel gemäß Anspruch 1, gekennzeichnet durch einen Gehalt an dem Oxycarbonsäureamid der Formel

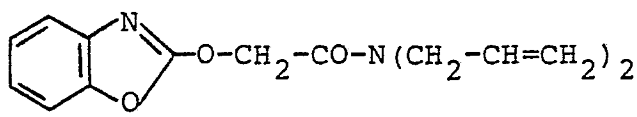

6) Mittel gemäß Anspruch 1, gekennzeichnet durch einen Gehalt an dem Oxycarbonsäureamid der Formel

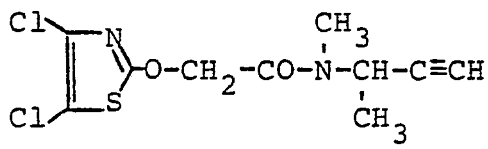

7) Mittel gemäß Anspruch 1, gekennzeichnet durch einen Gehalt an dem Oxycarbonsäureamid der Formel

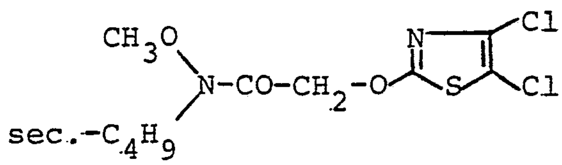

8) Mittel gemäß Anspruch 1, gekennzeichnet durch einen Gehalt an dem Oxycarbonsäureamid der Formel

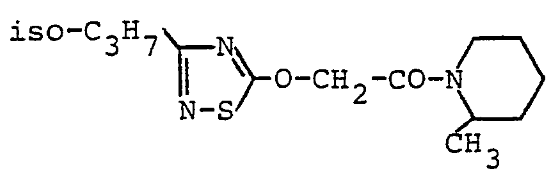

9) Mittel gemäß Anspruch 1, gekennzeichnet durch einen Gehalt an dem Oxycarbonsäureamid der Formel